# EUROPEAN PATENT APPLICATION

(11) **EP 4 818 696 A1**
(43) Date of publication of application: **07.10.2026**
(21) Application number: 24837680.8
(22) Date of filing: 13.11.2024
(51) Int. Cl.: G01N 33/00, G01N 1/22, G01N 1/02

(54) **METHOD FOR DETERMINING SOURCES OF POLLUTION**

(30) Priority: 30.11.2023 ES 202330994
(71) Applicant: Libelium Comunicaciones Distribuidas S.L., 50018 Zaragoza (ES)
(72) Inventor: JARA VALERA, Antonio Jesús, 30562 CEUTI (Murcia) (ES); CUEVAS MARTINEZ, Iris, 30562 CEUTI (Murcia) (ES); PUJANTE PÉREZ, Alejandro, 30562 CEUTI (Murcia) (ES); MERINO GARCÍA, Jorge María, 30562 CEUTI (Murcia) (ES); ILLUECA FERNÁNDEZ, Eduardo, 30562 CEUTI (Murcia) (ES); ASÍN PÉREZ, Alicia, 30562 CEUTI (Murcia) (ES)
(74) Representative: Herrero & Asociados, S.L.
(86) International application number: PCT/ES2024/070704
(87) International publication number: WO 2025/114623

(57) **Abstract**

A method for determining pollutant emission sources comprising an initial phase of collecting information from the study area, determining the ideal locations for sample collectors using chemical and meteorological models such as CHIMERE and WRF, analyzing the samples in an intermediate phase using techniques adapted to the pollutant, focusing on particle concentration, and analyzing in a final phase the data obtained and the information collected in the previous phases to identify and locate the pollutant sources. This analysis includes a statistical study of percentiles and meteorological conditions to detect significant exceedances in particle concentration, and an analysis using a positive matrix factorization model, PMF model, to break down the concentrations according to the pollution factors. This comprehensive approach allows to accurately determine the pollution sources and their specific contribution in the study area.

## Description

### OBJECT OF THE INVENTION

The technical field of the present invention is situated at the intersection of environmental engineering with a particular focus on the assessment and management of air quality in areas contaminated with suspended particulate matter of anthropogenic or natural origin.

In particular, the present invention relates to an automated method for determining point or dispersed sources of pollutants, which allows identifying, characterizing, locating and estimating the contribution of such polluting sources with the aim of evaluating the impact of sources of particulate or gaseous emissions in nearby urban centers. The method is especially focused on the study of dispersed and heterogeneous sources, such as open mining, landfills, agricultural farms, etc., located in an area of varying size situated on the periphery of the study site.

### BACKGROUND OF THE INVENTION

There are currently various studies related to the impact of particle-emitting activity areas. These activities can be diverse: among them, there can be industrial, agricultural, road traffic, natural sources or activities polluting the soil. For their study, various methodologies have been used with the aim of evaluating the extent and magnitude of the pollution. These include techniques to determine the best location of sampling devices and analysis of air, water and soil to identify and quantify the contaminants. These techniques actually have two sub-phases: in the first, the places with the greatest potential or gradient of "positive" or significant data are studied, and then in a second, by placing the collectors or analyzers in these areas, the sampling is carried out and non-zero data is obtained, which allows the modeling of the contaminants and the estimation of the impact. Epidemiological studies have also been used to evaluate the effects on the health of the exposed population, as well as computational modeling to predict the dispersion of contaminants and their scope in surrounding urban areas.

On the one hand, the CHIMERE chemical transport model is well-known and is widely accepted in the air pollutant modelling sector. CHIMERE is an open-source physical/chemical model designed to perform accurate analyses of atmospheric pollutant spread, pollution episodes, daily forecasts of ozone, aerosols and other pollutants, as well as long-term simulations (whole seasons or years) in emission control scenarios. In technical terms, CHIMERE is a multi-scale chemical transport model, from urban to continental, that allows the quantification over time of the evolution of air masses and pollution plumes (dispersions of pollutants in the air originating from a specific source). It is known that CHIMERE can be used as an air quality forecasting model, also integrating with meteorological models, which increases the accuracy of the results.

On the other hand, despite the advances, there are challenges and disadvantages in research of this type. One of the main limitations is the difficulty in establishing a clear line of causality between the emitting activities and pollution in the study site given the presence of other polluting factors and the variability in environmental and exposure conditions. In addition, the lack of adequate historical data and the complexity of quantifying long-term effects on human health and the environment represent significant obstacles to fully understanding the impacts. In addition, active participation and collaboration between public entities, industries, local communities and scientists are often challenging, which can limit the availability of data and the embodiment of effective mitigation measures. In this regard, transparency and collaboration are essential to comprehensively address the impacts of pollution from the different activities in urban centers or sensitive areas and work towards more effective and sustainable solutions.

Existing state-of-the-art solutions are generally limited to a retrospective analysis of historical data, without offering a detailed methodology to determine optimal sampling locations or to accurately identify and characterize air pollutant emission sources.

Therefore, the objective technical problem that arises is the lack of precision and specificity in the study of the embodiment of sampling points and the identification of sources of contaminants in the target areas, as for example in the case of an applied use related to mining activities.

### DESCRIPTION OF THE INVENTION

The present invention serves to solve the above-mentioned problem, through a computer-implementable methodology allowing a more accurate and adaptive determination of critical zones for the placement of air quality monitoring devices and a more precise characterization of pollution emission sources, including their type and contribution to the general profile of pollutants in the atmosphere. This method overcomes the challenges of conventional approaches by integrating advanced numerical simulation models and adapting existing sampling criteria, thus improving the effectiveness in mitigating the environmental impact of mining activity and in protecting public health.

One aspect of the invention relates to a method implemented to determine sources of pollutant emissions, which is defined according to claim 1. The dependent claims define advantageous embodiments.

Another aspect of the invention relates to a computer program product comprising instructions which, when the program is executed by a computer, cause it to carry out the method defined above.

Another aspect of the invention relates to a computer-readable medium comprising instructions which, when executed by the computer, cause it to execute the method defined above.

The advantages of the present invention over the prior art are fundamentally:
- Through a preliminary study of macro- and micro-embodiment of the proposed methodology, the shortcomings present in other existing studies are covered, where, although a background study is carried out to establish the layout of the sampling points, all are limited to an analysis of historical data or databases of the locations where there is a probability of being a potential source of emissions.
- The use of numerical simulation technologies and the use of a physical/chemical model of the propagation of pollutants in the atmosphere (for example, the CHIMERE model), with a specific configuration of the model, allows to narrow down and determine which is the optimal area for the placement of air quality devices, making an assessment of the areas most affected by the considered sources. In terms of micro-embodiment, criteria applied in sampling campaigns of other pollutants are adapted to adaptively combine them with the target pollutant of the study.
- The proposed methodology allows the identification of sources and to obtain not only which sources would be the emission sources but also the characterization of the sector from which they come. In contrast, other methods of source identification assess the contributions of pollutants by the emitters only approximately, identifying the most probable sources according to the nature of the pollutant and its concentration, or, breaking down the emission sources from the point of emission, that is, determining the dispersion of the pollutant, but already knowing previously what the emission source is. The types of identification methods already in existence use different approximations: i) either, the quantities and nature of the pollutants can be determined, but without knowing specifically the emission source; ii) or, the nature of the pollutant can be determined and therefore its dispersion, but assuming that the emission point is known.

These and other advantages can be derived in light of the description of the invention presented in detail below.

### DESCRIPTION OF THE DRAWINGS

To complement the description being made and in order to assist in a better understanding of the characteristics of the invention, in accordance with a preferred example of its practical embodiment, a set of drawings is included as an integral part of said description, in which, for illustrative and non-limiting purposes, the following has been represented:
Figure 1.- Shows an example of a diagram for sectorization by quadrants used in identifying the origin of the pollutant emission source.
Figure 2.- Shows a histogram of wind directions for environmental data analysis performed using positive matrix factorization.
Figure 3.- Shows in the diagram of sectorization by quadrants, trends in frequency and intensity of wind directions.
Figure 4.- Shows an image of a study area with the diagram superimposed of quadrants and wind directions and graphic signage of various areas of metal species for different wind speeds.
Figure 5.- Shows a histogram of the concentration percentages of each metal species obtained using a PMF model for a predominant wind direction.
Figure 6.- Shows in a heat map different concentration levels over a study domain resulting from a simulation of the CHIMERE model.

### PREFERRED EMBODIMENT OF THE INVENTION

Next is provided, with the aid of the figures referred to above, a detailed explanation of a preferred embodiment of the object of the present invention related to a methodology that uses different processes by which the emission sources of the target pollutant are determined. These processes are divided into different stages that range from the study of background information to the selection of sampling points up to the final phase where the emission sources are determined. These main stages are:
- Selection of sampling points
- Measurement and data extraction campaigns
- Determination and evaluation of sources

Below, according to a possible embodiment, the aforementioned stages of the method are detailed, that are divided into three main phases:
- A first phase of preliminary study and macro-deployment of sample collectors (or analyzers), for example, collectors of metals in the air.
- A second phase including the design of campaigns capable of covering a study area to collect the largest possible amount of data on the samples collected.
- A final phase corresponding to a post-processing of these collected data and a contribution study and determination of sources from the collected samples.

### I. First phase: Selection of sampling points

### a) Previous studies

A collection of information is carried out on the background of the entire area to be sampled, as well as information on the possible types of pollution, industry or other sources of emissions, geographical situation, meteorological conditions, etc., in said area. This first step is important since, depending on the potential sources of pollution found, they will be introduced into the CHIMERE model of propagation of pollutants described below.

### b) Macro-deployment

Macro-deployment refers to the study of the best locations for the placement of large-scale devices, that is, determining the location of the sample collectors (usually within a range of a few kilometers; this can be a town or a similar determined area). To do this, it is first necessary to know which areas are emitting metals into the atmosphere, to then use a physical/chemical model of contaminant propagation (in the example described here, the CHIMERE model) coupled with a meteorological model simulating the dispersion of particles suspended in the air, thus determining which are the most affected areas. In other words, "virtual" sources of pollution are created that have been previously detected in section a) and from which the behavior and propagation of metals in the air is studied, determining the areas most likely to collect pollution and, therefore, which are defined as the study area. This macro-deployment study ensures that the samples subsequently collected by the sampling teams are not biased by any polluting activity or situation that is not significant.

CHIMERE offers numerical simulation and prediction of the dispersion of particles and other pollutants through initial emission input data to subsequently perform zoning in order to detect clusters with similar particle concentration values that allow determining through principal component analysis (PCA) correlations the best location for the sample collectors.

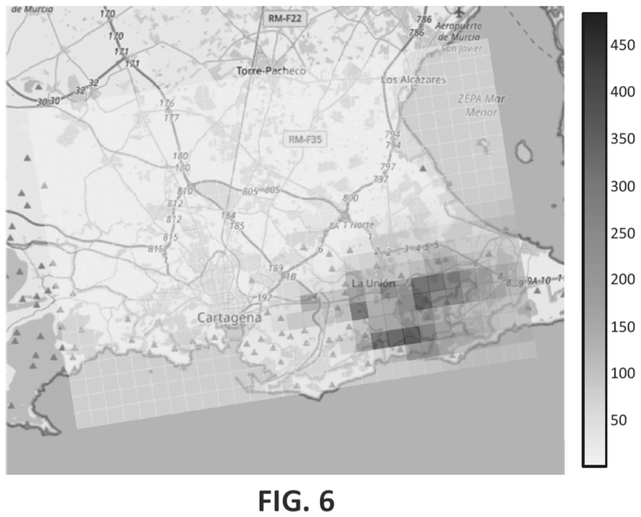

CHIMERE works in conjunction with different meteorological simulation models. In this case, the WRF model (WRF: "Weather Research and Forecasting") has been used, which represents the state of the art in numerical weather prediction (NWP). The WRF model is capable of making a prediction of meteorological variables such as wind, temperature or precipitation based on global meteorological files from NOAA (National Oceanic and Atmospheric Administration). In this phase, the emission of a potential source of mining origin can be simulated and how it spreads in space can be seen in order to define which areas receive more particulate matter. Figure 6, described below, shows an example of the representation of the defined areas.

In one possible embodiment, a simulation is run for the hours specified in a file ("script" in "bash") containing a series of commands and control structures executed by a Unix-based operating system and using a command interpreter providing an interface between the user and the operating system. Depending on the study to be carried out, a domain is defined, which is the geographic area of study that is to be covered along with the emissions scenario in that geographic area. The study domain and the one that CHIMERE works with is defined in different cells, units of minimum space on which the results are obtained. This marks the resolution of the model; in this case, for example, cells of 1x1 km have been selected.

In order to simulate particle emissions from sources or foci, 'artificial' emissions are generated. To do this, first of all, all types of emissions related to gases are eliminated, to prevent the model from taking into account chemical reactions in the atmosphere that are not of interest. In this way, only particle emissions that do not react chemically in the atmosphere are generated, i.e. they are direct emissions, not immissions (generated by gases such as volatile organic compounds or VOCs). The most important direct emissions are black carbon (BC) and organic carbon (OC). Therefore, these particles behave similarly to metallic particles since they are only affected by diffusion and transport processes. If potential emission points are generated from mining activities of BC and OC in the locations studied, they move like metallic particles, so that the transport of particulate material is only correlated with the distribution of metallic particles. Artificial emissions are generated based on the position, size, activity, metals present in the areas studied, as well as any other relevant prior information. Artificial emissions are generated based on a multiplier of the real emissions, extracted from available, inventoried or satellite databases, in this case from the CAMS (Copernicus Atmosphere Monitoring Service) inventory and increased in an extraordinary way in those boxes or positions that coincide with the emissions, improving the total gradients for better identification. The area of the mining operation is proportional to the increased emissions. This means creating a specific and extraordinary configuration of the model that allows the maximum performance to be obtained from it.

In a possible simulation example, the emission type is PM10 particles (with an aerodynamic diameter of less than 10 µm - micrometers), using BC particle pollutants as a basis. These particles are defined as ultrafine particles, smaller than PM1 (polluting particles of less than 1 micron), but the simulation forces particles between 0 and 10 µm to be generated, based on the composition and stability of BC particles, in order to model transport.

The next step is to simulate the particle emission from the study areas. These emissions are assigned to each of the cells (for example, 10x10 km cells) taking into account the study area within the cell so that the emission is proportional to the size of the study area. To do this, the locations of the shape files of the studied areas defined in the previous section are taken into account, to assign them to a cell and see the temporal evolution of the concentration on the mesh.

### c) Micro-deployment

The aim of the micro-deployment study is to determine exactly where the pollutant is to be sampled, that is, to determine the suitability of the location where the sampling device is to be located. In the case of particles, we speak of sample collection, and the collectors can be differentiated into High Volume (HV), Low Volume (LV) and sedimentable collectors. In the case of gases, gas analyzers, portable cabinets, collection in solid or gelled media, or Tedlar bags for subsequent analysis in the laboratory can be used as sampling devices.

For example, the following criteria are used when selecting the locations of the collection devices:
- Flow restriction: The accumulation of contaminants due to environmental characteristics (buildings, topography, etc.) that hinder air circulation must be avoided, thereby preventing the measurement of low-dispersion microenvironments.
- Height: The building has a suitable area to enable the device, between 1.5 and 4 meters to improve air quality.
- Free arc 270°/180° (≤3m): At the installation site, the device has a clear angle where it can collect data in the most efficient way possible.
- Obstacles (≤6m): There are no obstacles within the vicinity of 6 meters, thus avoiding obstruction by trees, street lighting, structures, etc., to the extent possible.
- Proximity to highway and heavy traffic: The emission of particles by road traffic can affect the capture of target particles, which is why locations are chosen away from roads with the highest traffic intensity.
- Contaminant trajectories: These simulations performed with CHIMERE are used as determining factor when rejecting or prioritizing an area under study.
- Safety conditions for the equipment: Locations are sought where the equipment has a safe environment that guarantees the integrity of the devices against vandalism, taking into account the availability of fencing, security cameras, etc.
- Access for inspections and sampling: It is valued positively if the environment where the device is located is accessible to the technician and facilitates the taking of samples as much as possible.

Based on macro- and micro-deployment criteria, a numerical evaluation system is established for the locations of collectors for particle measurement, with the aim of choosing the best location for the installation of high and low volume collectors (AV, BV) and sedimentable tanks.

### II. Second phase: Measurement campaigns according to reference technique

Data collection, an intermediate phase of the methodology, from measurements made by collectors (for example, collectors of settling particles), can be implemented using different techniques that depend on the nature of the air pollutant whose emission source needs to be determined. For example, for a project to determine particle emission sources, measurement campaigns can be divided into main groups, one for each type of collector, BV or low volume campaigns, AV or high-volume campaigns and settling campaigns. Due to the nature of the samples to be identified, for example, metals, the samples are analyzed with a pretreatment of the collector filters that consists of washing with distilled water, stabilization, weighing and identification that ensures the traceability of the samples. For example, for the determination of metals in the soluble fraction, the samples must be acidified upon arrival at the analysis laboratory, while, for the determination of metals in the insoluble fraction, the total sample volume is filtered on a cellulose acetate filter. The analytical results are the input to the last phase of the method to determine the emission sources of the target pollutant.

### III. Final phase: Determination of sources

The identification and contribution of sources is the ultimate goal of processing the laboratory results, based on the data obtained from sampling using the different techniques in the previous phase (analytical results). Two methodologies are applied to analyze the analytical results: the first is the statistical study of percentiles and base meteorological conditions, the second consists of simulations using the statistical software Positive Matrix Factorization or PMF, which is a tool designed to analyze and decompose environmental data, especially in the context of air quality. Finally, both data are combined to discern between sources that contribute to the samples.

### i) Statistical analysis of percentiles and base meteorological conditions

For the first method of analysis of the analytical results, the exceedances of the 90th percentile for each metal species are identified and grouped based on established criteria in the division of quadrants by wind directionality. With this, together with the wind speed, retro-trajectories can be traced that lead to the possible metal emission sources.

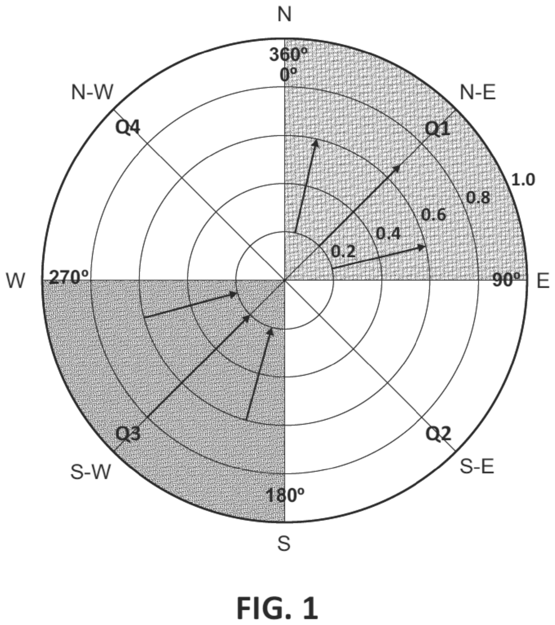

To identify by quadrants exceedances of the 90th percentile (anomalous emissions) of each metal species for each location, only the data collected in high-volume measurement campaigns are used, in the locations where they have been carried out. And in the locations where there are no such campaigns, low-volume campaigns with a narrower spectrum are used. Once the data have been identified, these percentiles are broken down using the following criterion: they are divided into four quadrants (four for each location), each quadrant corresponding to a degree range referring to the wind directional angles downwind of the collector. These quadrants, illustrated in Figure 1, are named as:
**Q1**: >0° to ≤ 90°, corresponds to a northeast direction **NE**
**Q2**: >90° to ≤ 180°, corresponds to a southeast direction **SE**
**Q3**: >180° to ≤ 270°, corresponds to a southwest direction **SW**
**Q4**: >270° to ≤ 360° (0°), corresponds to a northwest direction **NW**

The quadrant diagram example shown in Figure 1 is based on a compass rose indicating the cardinal points and the diagram implementable in a graphical interface shows:
- Degrees: Indications around the entire circumference near the outer edge, indicating the degrees belonging to the intervals into which the quadrants are divided.
- Quadrants **Q1, Q2, Q3** and **Q4**. In the example, the quadrant **Q1** to the lee of the collector, is the direction in which the contaminants are detected to be dispersing (represented with arrows towards the outside of the circumference); while the quadrant **Q3** indicates the direction in which the emitting sources are located, in this case, upwind of the collector.
- Arrows: Example of representation of information to visually appreciate the direction of dispersion of particles according to the prevailing winds.

In this way, the exceedance of the 90th percentile in a specific direction is associated to trace a retrotrajectory and identify the origin of the emitting source. In addition, a wind speed criterion is added to further break down the samples. This criterion separates the exceedances into two groups within the quadrant, the first group as exceedances that only occur when there is a wind speed greater than or equal to 3 m/s and the second group in which the exceedances that only occur where the wind is less than 3 m/s are collected. The resulting difference places the emitting sources at a greater or lesser degree of proximity, with distant sources being understood as those that give rise to percentile exceedances where the wind speed is ≥3 m/s and nearby sources where it is <3 m/s. In some cases, it is complemented with correlation matrices to find groups of metals with the same origin.

### ii) Positive Matrix Factorization Analysis

The simulation part using the PMF model collects the metal concentration signals from each of the collectors and separates them into different factors contributing to these results through statistical methods and linear regression adjustments. This model is fed, in addition to the actual concentrations of contaminants collected by collectors, by the uncertainties of the measurements carried out.

Conducting a source contribution analysis on air quality is an essential aspect for understanding and addressing environmental pollution problems, especially in regions close to industrial and mining activities. In this context, the EPA PMF 5.0 (Environmental Protection Agency - Positive Matrix Factorization) model is specifically used as a tool to estimate the contribution of different sources to the concentration of air pollutants. This model unravels complex mixtures of atmospheric particles and provides quantitative identification of sources, giving insight into the impacts of mining activities on air quality and ultimately on human health and the environment.

Particulate collectors, in this case high- and low-volume collectors and settling particle filter collectors, provide accurate data on metal concentrations in the air. However, directly interpreting these data can be difficult due to the complexity of the mix of air pollution sources in urban and industrial areas. Therefore, the aim of using this model is to discern which of the samples collected by the collectors come from mining operations and which are due to other factors. This includes differentiating between direct emissions from mines, natural sources or sources from industry. To do this, the PMF model is fed with two types of input data: concentration measurements of the samples (metals) collected by the collectors for each of the locations and the uncertainties associated with each of these measurements. The PMF model assumes that there are p sources, source types or source regions, called factors, that influence the receptor and that, in addition, the concentrations observed for the different sources have been produced by linear combination of the impacts of the p factors. The mathematical expression that describes the final concentration is as follows: ${x}_{ij} = {\sum }_{k = 1}^{p} {g}_{ik} ⋅ {f}_{ij} + {e}_{ij}$ where xᵢⱼ is the concentration in the receptor for the j species on day i, gᵢₖ is the contribution of the k factors on day i, fᵢⱼ is the fraction of the k factors that is species j, and finally eᵢⱼ is the residual of species j on day i.

That is, in this model it is assumed that only the concentrations xᵢⱼ are known and the objective is to find the contributions gᵢₖ and the fractions fᵢⱼ. To do this, the PMF model uses the least squares technique to minimize the difference between the concentration data and those obtained through the weights g and f. The aim is to minimize the following equation: $Q = {\sum }_{i = 1}^{n} {\sum }_{j = 1}^{m} \left(\frac{{x}_{ij} - {\sum }_{k = 1}^{p} {g}_{ik} - {f}_{ij}}{{s}_{ij}}\right)$ where sᵢⱼ is the uncertainty of the j species for day i.

As a final result, the PMF model returns the percentage contribution of each of the factors to the samples provided.

Before running the model, the model configuration is set to indicate the number of factors into which the concentration of the samples is to be broken down, which in a possible case could be broken down into the following factors:
- Factor A: Industrial
- Factor B: Combustion gases (road traffic, biomass combustion, etc.)
- Factor C: Potential sources of mining origin
- Factor D: Others, such as background (urban, agricultural or whatever is relevant to the area studied)
- Factor E: Calima or other extraordinary natural sources

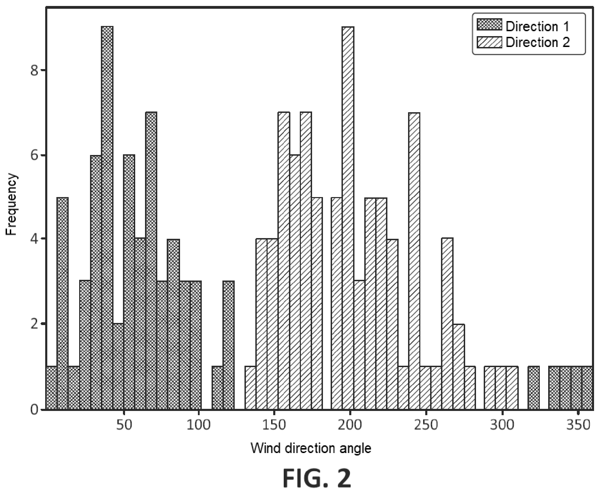

After compiling all the historical data collected by the collectors in each of the campaigns, the PMF model is run for each site, thus obtaining the contribution of each of the factors to each species. In addition, the PMF model simulations are distinguished by sampling technique, i.e. high volume and low volume. For example:
- First case: using the entire historical record. The PMF model is run with the entire historical record of samples collected for each location, separating between high and low volume. The PMF model returns the contribution percentages of each of the sources to each metal.
- Second case: data filtered by wind direction. Additionally, and since wind is one of the variables that plays an important role in the transport of pollutants in the atmosphere, the samples are divided according to the predominant wind direction. At each location, 50%-50% of the samples are divided. A meteorological study of the two most predominant wind directions is made to subsequently divide the data set of species concentrations into two equal subsets, assigning each value to one or another wind direction according to the day to which they belong. This analysis helps to determine the type of each of the factors that PMF shows in the simulation. Figure 2 shows, in a histogram, the separation of the wind directions into two from the predominant directions for each of the locations, representing the frequency and angle of the wind for each of the two predominant wind directions.

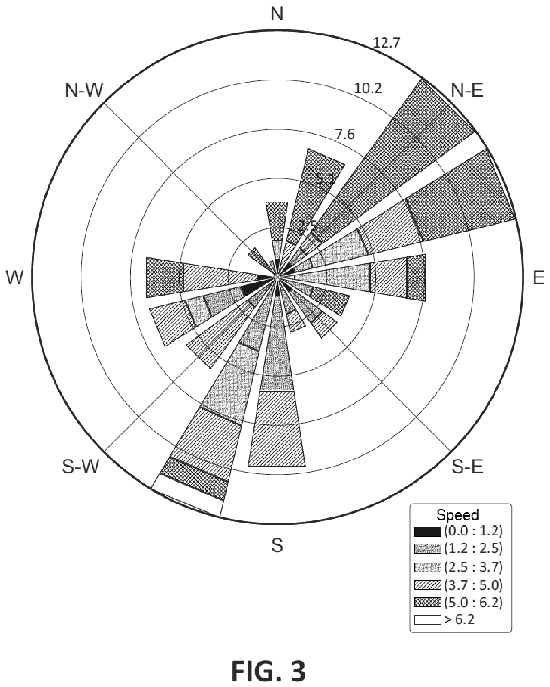

Figures 3-5 below show results based on an example of the implemented methodology that uses data resulting from measurement campaigns that are identified in the Figures with the following terminology:
- AV: Data collected through high volume campaigns
- BV: Data collected through low volume campaigns
- MIXED: Data collected through low-volume campaigns, which have been considered insufficient to carry out PMF executions, so they have been completed (same location and not coinciding in time) with data from high-volume campaigns carried out in the same location and date.

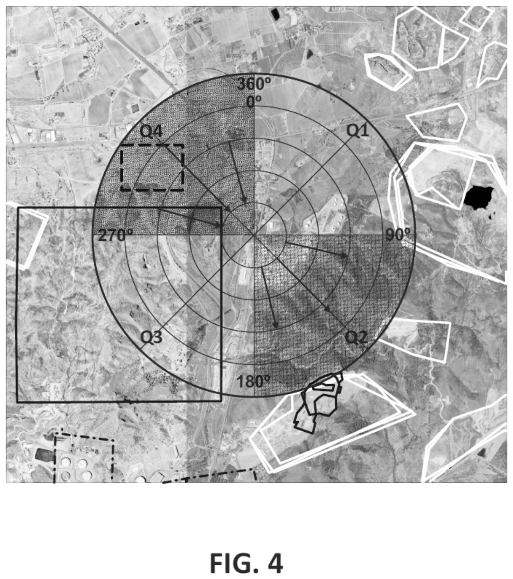

The methodology, according to an example, is based on the following steps to determine the factors:
1. The data corresponding to each location is divided: data from the AV, BV and MIXED sensors, meteorology, including wind speed and direction. This information is then divided into four quadrants (**Q1** from 1° to 90°, **Q2** from 91° to 180°, **Q3** from 181° to 270° and **Q4** from 271° to 360°). The position from which the directionality data with respect to metal pickup was taken is always taken into account to determine the quadrant.
   For example, in Figure 3 the frequency and intensity of wind directions in the North (N), South (S), East (E) and West (W), or in degrees of North azimuth (0-360°), are represented in a wind rose for a specific area:
   - 0° = North (North wind)
   - 45° = Northeast (Northeast wind)
   - 90° = East (East wind)
   - 135° = Southeast (Southeast wind)
   - 180° = South (South wind)
   - 225 ° = Southwest (Southwest wind)
   - 270 ° = West (West wind)
   - 315° = Northwest (Northwest wind)
   - 360° = North
   Wind data uses a projection of angles other than the algebraic (traditional) one, with North always being the origin of the coordinates, so it is important to take this into account based on wind projections.
2. For each quadrant, the metals of the exceedances (90th percentile) are grouped together, breaking down into two large groups: metals transported by wind speeds below 3 m/s and metals transported by wind speeds greater than 3 m/s (This value of ≥3 or <3 m/s is an example of a value where a difference has been observed between the metal groupings). This separation determines the approximate proximity of the emission source, i.e. particles transported by winds below 3 m/s are located closer to the collection site than particles transported by higher wind speeds.
   For example, Figure 4 shows an image of a given study area with the compass rose superimposed on the image representing the following parameters and results:
   Predominant wind direction: SE, quadrant **Q2**.
   Possible sources emitting from direction: NW, quadrant **Q4**.
   Species that only occur with wind speeds greater than 3 m/s, represented in the areas outlined in black: Aluminum, Barium, Phosphorus, Iron, Potassium, Titanium, Zinc and Sodium.
   Species that only occur with wind speeds less than 3 m/s, represented in the areas outlined in white: Lead and Sulfur.
   In this example, it is thus deduced that using the collection of information on the background of the sampled area, since lead and sulphur are present in nearby transport winds, it is possible that most of these two metals are due to an existing mine in the area and that the rest of the metals are of industrial origin due to the companies located in the area and the agricultural sector.
3. Using the results obtained from the PMF model, the percentages of each metal associated with each factor are checked, analyzing the correlation between metals and factors, to determine which metals have a common origin. These analyzed results have been previously broken down into two predominant directions for each location, using 50% of the samples in each one. For quadrants where there are no results of predominant winds broken down by PMF, PMF results without breaking down by predominant direction (all samples) can be used. In addition, raw data of the possible sources and directions of the wind are used.
4. To determine the possible sources, the division of factors is used in the case of PMF and, if the quadrant does not coincide, the sources present in the area. For this determination, some of the possible factors are the following:
   a. Composition of potential sources from mining activity that falls within the possible wind trajectories (upwind of the collector and downwind of said source).
   b. Composition of industrial emissions, extracted from the PRTR (RETC registers for their acronym in Spanish, or PRTR records for its acronym in English "Pollutant Release and Transfer Registers").
   c. Episodes of intrusion of Saharan dust or other natural sources.
   d. Emissions from agricultural and livestock sources.
   e. Other background emissions of combustion gases (traffic, boilers, etc.)

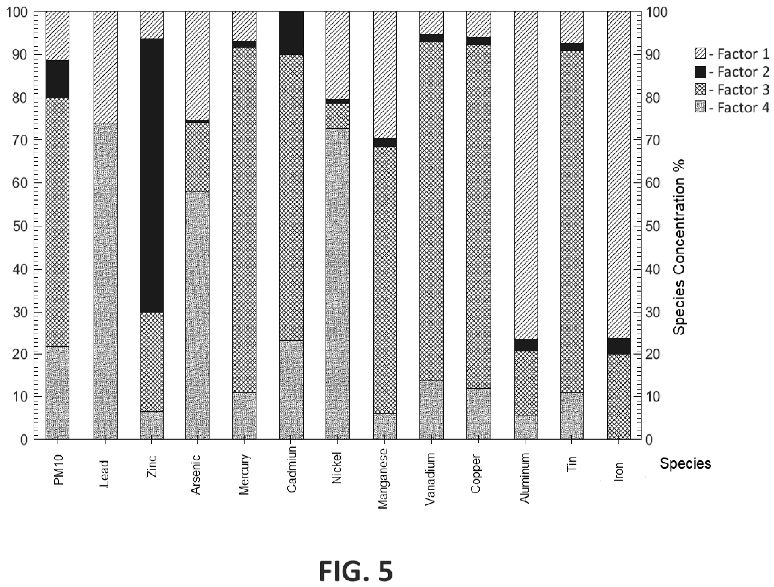

For example, Figure 5 represents in a histogram the results of the PMF model, considering one of the two predominant wind directions and the sample data from high volume (AV) collectors, obtaining the concentration percentages of the possible species for four factors:
Factor 1 - Agricultural
Factor 2 - Industry - There is a grouping of metals of industrial origin
Factor 3 - Potential sources for mining activity
Factor 4 - Undetermined

Finally, Figure 6 shows a heat map of the emissions simulated with the CHIMERE model; in the example, referring to lead concentrations. Heat maps can be obtained for each mineral exploitation, with a kilometric resolution, composing the main results of the macro-deployment study. This information is key in determining the "target", reference point or background pollution of the study area.

The method described is equally applicable to gaseous emissions, modifying only some data sources and adapting the field sampling methods.

## Claims

1. A computer-implemented method for determining emission sources of a target pollutant, **characterized by** comprising the following steps executed by one or more processors:
- in a first phase,
- collecting background information from one or more sample zones within a study area, the background information including pollution types, industry, geographic situation, and meteorological conditions given in each sample zone;
- determining at least one location of one or more sample collectors in each zone to be sampled to cover the study area according to macro-deployment criteria, wherein the at least one location is determined using a combination of chemical and meteorological models configured to predict a dispersion of particles of the target contaminant in the study area;
- determining, based on micro-deployment criteria, a suitability value for each location determined in the previous step for each sample collector and choosing the location with the highest suitability value;
- in a second phase,
- obtaining data from samples collected at a location chosen by the one or more sample collectors in each area to be sampled, wherein the data are obtained by analytical techniques adapted to the target pollutant applied to the collected samples, the data obtained comprising at least one measurement of particle concentration of the target pollutant; and
- in a third phase,
- analyzing the data obtained in the previous phase to determine an identification and location of emission sources of the target pollutant using the background information collected in the first phase and estimating a contribution of each source to the particle concentration measurement of the target pollutant obtained in the second phase, wherein the data are analyzed by two procedures: i) a statistical analysis of percentiles and meteorological conditions to identify exceedances of the 90th percentile in the particle concentration prediction, and ii) a positive matrix factorization analysis to separate the particle concentration predictions according to factors contributing to pollution in the study area.

2. The method according to claim 1, wherein the chemical model used to determine the location of the one or more sample collectors is the CHIMERE model configured to identify regions based on particle concentration.

3. The method according to claim 2, wherein the CHIMERE model is coupled with an atmospheric simulation climate model which is the WRF model.

4. The method according to any of the preceding claims, wherein high volume, low volume and settleable sample collectors are used.

5. The method according to any of the preceding claims, wherein the statistical analysis of percentiles and meteorological conditions comprises an analysis by quadrants based on the directionality of the wind to obtain particle retrotrajectories that determine the location of emission sources.

6. The method according to any of the preceding claims, wherein the positive matrix factorization analysis uses the particle concentration measurement of the target pollutant and an uncertainty value of the measurement obtained in the second phase.

7. The method according to any of the preceding claims, wherein the positive matrix factorization analysis uses the target pollutant particle concentration measurement filtered by wind direction.

8. A computer program product comprising instructions that, when the program is executed by a computer, cause the computer to carry out the method of claims 1-7.

9. A computer-readable medium comprising instructions that, when executed by a computer, cause the computer to carry out the method of claims 1-7.
